(19) Europäisches Patentamt
European Patent Office
Office européen des brevets

(11) Veröffentlichungsnummer: **0 168 661**
**B1**

(12) # EUROPÄISCHE PATENTSCHRIFT

(45) Veröffentlichungstag der Patentschrift: **24.08.88**

(51) Int. Cl.[4]: **C 07 D 231/46**, G 01 N 31/22 // C12Q1/28

(21) Anmeldenummer: **85107564.8**

(22) Anmeldetag: **19.06.85**

(54) **Neue Aminopyrazolinone, ihre Herstellung und Verwendung.**

(30) Priorität: **19.06.84 DE 3422732**

(43) Veröffentlichungstag der Anmeldung:
**22.01.86 Patentblatt 86/4**

(45) Bekanntmachung des Hinweises auf die Patenterteilung:
**24.08.88 Patentblatt 88/34**

(84) Benannte Vertragsstaaten:
**AT BE CH DE FR GB IT LI LU NL SE**

(56) Entgegenhaltungen:
**DE - A - 3 100 807**
**DE - C - 92 009**
**US - A - 4 321 397**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Batz, Hans-Georg, Dr., Traubinger Strasse 63, D-8132 Tutzing (DE)**
Erfinder: **Herrmann, Rupert, Dr., Heinrichstrasse 3, D-8120 Weilheim (DE)**
Erfinder: **Siedel, Joachim, Dr., Bahnhofstrasse 6, D-8131 Bernried (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 168 661 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Aminopyrazolinone, ihre Herstellung und Verwendung als Farbkuppler zum $H_2O_2$-Nachweis in Gegenwart von Peroxidase und einer phenolischen oder anilinischen Kupplungskomponente.

Die analytische Bestimmung von enzymatisch – z. B. durch Oxidation eines Serumbestandteils mit Sauerstoff und einer Oxidase – gebildeten $H_2O_2$, hat für die medizinische Diagnostik eine erhebliche Bedeutung. Die Menge des gebildeten $H_2O_2$'s, die proportional der Menge des vorliegenden Oxidasesubstrats ist, lässt sich beispielsweise enzymatisch bestimmen, indem ein Chromogen, in Gegenwart von Peroxidase, mit $H_2O_2$ zu einem photometrisch bestimmbaren Farbstoff reagiert. Die Menge des gebildeten Farbstoffs dient als Mass für die Menge des $H_2O_2$-bildenden Substrats.

Ein bekanntes Chromogen zur $H_2O_2$-Bestimmung ist das Indikatorsystem nach Trinder (Ann. Clin. Biochemistry, 6, 24–27 (1969)), bei dem ein Farbkuppler (4-Aminoantipyrin, 4-AAP) mit einer Kupplungskomponente (Phenol) in Gegenwart von Peroxidase unter Einwirkung von $H_2O_2$ oxidativ zu einem Farbstoff gekuppelt wird. Anstelle des Phenols können auch andere phenolische oder anilinische Verbindungen verwendet werden, wobei die mit anilinischen Verbindungen, wie beispielsweise N-Ethyl-N-hydroxyethyl-3-methylanilin (EHT, DOS 31 24 594) oder N-Ethyl-N- (3-methylphenyl)-N'-acetamido-ethylendiamin (EMAE, EP-A-0 007 787) gebildeten Farbstoffe einen höheren molaren Extinktionskoeffizienten, bei einem um bathochrom ca. 50 nm verschobenen Wellenlängenmaximum, aufweisen.

Daraus ergibt sich, besonders bei der Bestimmung von niedrigkonzentrierten Serumbestandteilen wie z. B. Harnstoff und Creatinin, für die Verwendung von anilinischen Kupplungskomponenten gegenüber den phenolischen ein Vorteil.

Als Farbkuppler können, ausser 4-Aminoantipyrin, auch andere Aminoantipyrinderivate (wie z. B. Diaminoantipyrin, DE-OS 31 00 807) Methylbenzthiazolonhydrazon (MBTH), sulfoniertes MBTH (SMBTH) und ähnlich reagierende Verbindungen eingesetzt werden. Ein Nachteil all dieser Farbkuppler bei der oben beschriebenen $H_2O_2$-Bestimmung, besteht, neben der durch Autoxidation bedingten teilweise schlechten Leerwertstabilität, in der mangelhaften Stabilität des mit anilinischen Kupplungskomponenten gebildeten Farbstoffs (s. z. B. DOS 31 24 549). Eine Verbesserung der Farbstabilität würde einen grossen Praktikabilitätsvorteil, z.B. durch Ermöglichung längerer Ablesezeiten, bedeuten.

Der Erfindung liegt daher die Aufgabe zugrunde, neue Verbindungen zu schaffen, welche die oben angegebenen Nachteile nicht aufweisen und als Farbkuppler bei der $H_2O_2$-Bestimmung anstelle von 4-Aminoantipyrin oder anderen bekannten Farbkupplern verwendet werden können und die insbesondere eine bessere Farbstabilität der bei ihrer oxidativen Kupplung mit anilinischen Kupplungskomponenten gebildeten Farbstoffe aufweisen.

Gelöst wird diese Aufgabe erfindungsgemäss durch neue Aminopyrazolinone der allgemeinen Formel

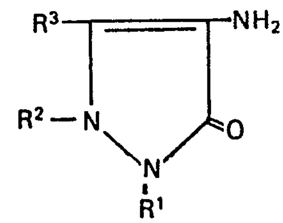

in der $R^1$, $R^2$ und $R^3$ geradkettige oder verzweigte Alkylgruppen mit 1 bis 6 C-Atomen darstellen und zusammen höchstens 8 C-Atome enthalten.

Die Herstellung der erfindungsgemässen Verbindungen kann beispielsweise durch Nitrierung oder Nitrosierung der entsprechenden Trialkylpyrazolinone nach an sich bekannten Methoden und anschliessende Reduktion der Nitro- oder Nitrosogruppe mit beispielsweise Zn/HCl oder Wasserstoff Katalysator erfolgen. Die bei dieser Synthese als Ausgangsmaterial dienenden Trialkylpyrazolinone erhält man nach ebenfalls bekannten Methoden z. B. durch Umsetzung von 1,2-Dialkylhydrazinen mit Acylessigester oder durch Alkylierung geeigneter Dialkylpyrazolinone. Derartige Methoden sind z. B. in W. Krohs, O. Hensel, Pyrazolone und Dioxopyrazolidine, Editio Cantor, Aulendorf/Wttbg., 1961 und R.H. Wiley und P. Wiley in A. Weissberger (Ed.), Pyrazolones, Pyrazolidones and Derivates, Interscience Publishers, New York–London–Sydney. 1964, beschrieben.

Bevorzugt wird als erfindungsgemässe Verbindung 4-Amino-1,2,3-trimethylpyrazolin-5-on (TAP). Beispiele für andere erfindungsgemässe Verbindungen sind 1,2,3-Triethyl-, pyrazolin-5-on, 1,2-Methyl- 3-isopropyl-pyrazolin- 5-on, 1-Butyl- 2,3-diethylpyrazolin-5-on, 1-Tert.-butyl- 2,3-dimethyl-pyrazolin-5-on, 1-Hexyl-2,3-dimethyl-pyrazolin-5-on usw.

Ein weiterer Gegenstand der Erfindung ist die Verwendung der obigen Verbindungen bei der $H_2O_2$-Bestimmung durch oxidativen Farbbildungsreaktion mit anilinischen oder phenolischen Komponenten und Peroxidase als Farbkuppler.

Als anilinische Kupplungskomponenten werden N-Ethyl- N-hydroxyethyl- 3-methylanilin (EHT) und N-Ethyl-N-(3-methylphenyl)-N'-acetamido-ethylendiamin (EMAE) bevorzugt. Andere im Rahmen der erfindungsgemässen Farbbildung geeignete anilinische Kupplungskomponenten sind N-Ethyl-N-(4-fluor-3-methylphenyl)- N'-acetamido-ethylendiamin (F-EMAE), N-Ethyl- N-(2-sulfoethyl)-m-toluidin (EST), N- Ethyl-N-(3'-sulfobenzyl)-m-toluidin, andere Anilinderivate, Naphthylamin, Naphthylaminderivate, Aminochinoline usw.

Die Farbbildung der bevorzugten erfindungsgemässen Verbindung 4-Amino-1,2,3-trimethylpyrazolin-5-on (TAP) als Farbkuppler mit EHT als anilinische Kupplungskomponente zeigt nachstehende Reaktionsgleichung:

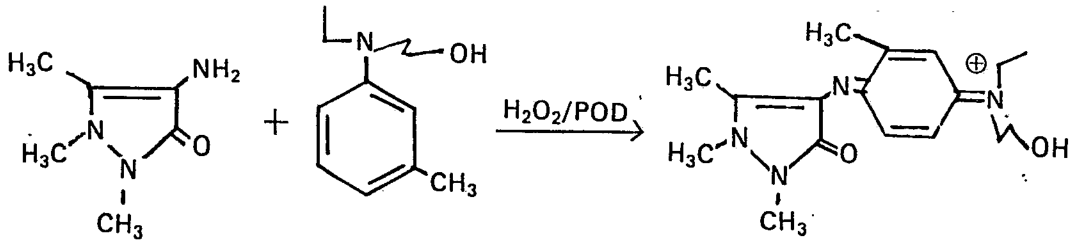


In Analogie zu der obigen Gleichung verläuft auch die Farbbildung der erfindungsgemässen Verbindungen mit beispielsweise phenolischen Verbindungen. Prinzipiell sind alle Verbindungen, die als Kupplungskomponenten bei oxidativen Farbbildungsreaktionen mit gebräuchlichen Kupplern eingesetzt werden, auch für die erfindungsgemässen Kuppler geeignet.

Beispiele für andere gebräuchliche Kupplungsverbindungen, die nicht zu den anilinischen Kupplungskomponenten gehören und zusammen mit den erfindungsgemässen Verbindungen zur Farbbildung im Rahmen des $H_2O_2$-Nachweises eingesetzt werden können sind Phenol, 4-Chlorphenol, andere Phenolderivate, Naphthol, Naphtholderivate, Hydroxychinoline, Dihydroxyphenylessigsäure usw.

Die Umsetzung bei der Farbstoffbildung wird in gepufferter Lösung durchgeführt. Als Puffersubstanzen und pH-Werte eignen sich die für Peroxidase bekannten diesbezüglichen Bedingungen. Bevorzugt werden pH-Werte zwischen 5 und 9. Im übrigen ist die Wahl des Puffers und des pH-Werts im Falle einer vorgeschalteten $H_2O_2$ bildenden enzymatischen Reaktion vor allem durch die Anforderungen hinsichtlich Puffer und pH-Wert der daran beteiligten Enzyme bedingt.

Diese Bedingungen sind sämtlich dem Fachmann bekannt und bedürfen daher hier keiner näheren Erläuterung.

Ein Reagenz zur Bestimmung von $H_2O_2$ auf Basis Peroxidase, wenigstens einer erfindungsgemässen Verbindung, wenigstens einer Kupplungskomponente und Puffersubstanz kann zusätzlich noch übliche Lösungsmittel, Stabilisatoren oder/und oberflächenaktive Substanzen enthalten. Besonders geeignet erwiesen sich dabei folgende Mengenverhältnisse der essentiellen Bestandteile dieses Reagenz:

0,1 bis 100 U/ml Peroxidase,

0,01 bis 20 mMol/l erfindungsgemässe Verbindung,

0,1 bis 50 mMol/l Kupplungskomponente.

Oberflächenaktive Mittel werden, wenn sie zugegen sind, vorzugsweise in Mengen von 0,001 bis 0,1 g/ml, bezogen auf gebrauchsfertige Reagenzlösung, eingesetzt.

Die folgenden Beispiele erläutern die Erfindung weiter.

Beispiel 1

Herstellung von 4-Amino-1,2,3-trimethylpyrazolin-5-on (TAP)

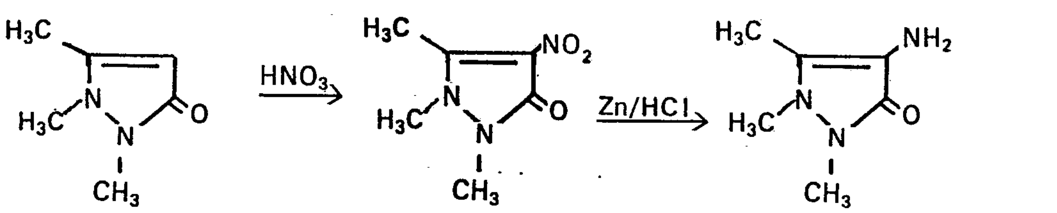


a) 4-Nitro-1,2,3-trimethylpyrazolin-5-on

Zu 5,5 ml konzentrierter Salpetersäure gibt man bei 70 °C portionsweise 1 g 1,2,3-Trimethylpyrazolin-5-on x $H_2O$. Die Temperatur soll dabei nicht über 75 °C steigen. 30 Minuten nach Beendigung der Zugabe wird die Reaktionsmischung im Vakuum eingeengt und auf 10 g Eis gegeben. Mit 5 N Natronlauge wird unter Kühlung auf pH 7 gestellt und das dabei ausgefallene Rohprodukt abgesaugt. Aus der Mutterlauge kann durch Einengen weiteres Produkt gewonnen werden. Durch Umkristallisation aus Wasser/Isopropanol wird das erhaltene rohe Nitropyrazolinon gereinigt.

Ausbeute: 0,96 g=71% d. Th.

$^1H$-NMR (DMSO-$d_6$): $\delta$=260 (s, 3H); 3,36 (s, 3H); 3,65 ppm (s, 3H).

Elementaranalyse: ($C_6H_9N_3O_3$, 171,16)
ber.: C 42,11 H 5,30 N 24,55
gef.: C 42,2 H 5,42 N 25,18

b) 4-Amino-1,2,3-trimethylpyrazolin-5-on

Zu einer Suspension aus 6,4 g Zinkstaub in 5 ml Wasser tropft man unter Kühlung eine Lösung von 2,8 g 4-Nitro-1,2,3-trimethylpyrazolin-5-on in 8,4 ml konzentrierte Salzsäure und 3 ml Wasser zu. Die Reaktionstemperatur wird dabei zwischen 25 und 30 °C gehalten. Bei 60 °C wird noch 2 Stunden nachgerührt, anschliessend das Reaktionsgemisch auf Eis gegeben und mit 5 N Natronlauge alkalisch gestellt. Die ausgefallenen Zinksalze werden abgesaugt, die Mutterlauge weitgehend eingeengt und mehrmals mit Chloroform ausgeschüttelt. Nach dem Trocknen der organischen Phase über Natriumsulfat wird im Vakuum eingedampft, wobei das gewünschte Produkt kristallisiert.

Ausbeute: 0,93 g=40% d.Th.

$^1$H–NMR (DMSO–$D_6$): $\delta$=1,98 (s, 3H); 2,85 (s, 3H); 3,11 (s, 3H) 3,63 ppm (s, br, 2H).

Elementaranalyse: ($C_6H_{11}N_3O$, 141,17)

ber.: C 51,09 H 7,85 N 29,76

gef.: C 49,56 H 7,93 N 28,9

Massenspektrum: m/e=141 ($M^+$)

Beispiel 2

Farbstabilitäten bei der oxidativen Kupplung verschiedener Kupplungskomponenten mit TAP, sowie mit 4-AAP als Vergleichssubstanz.

Konzentrationen im Testansatz:

| | |
|---|---|
| K–$PO_4$-Puffer, pH 7,0: | 0,1 mol/l |
| Farbkuppler: | $3\times10^{-2}$ mmol/l |
| Kupplungskomponente: | $3\times10^{-1}$ mmol/l |
| Triton X 100: | 5 mg/ml |
| Peroxidase: | 1,2 U/ml |
| Wasserstoffperoxid: | $1,5\times10^{-2}$ mmol/l. |

In der Vergleichsküvette befindet sich Puffer. Die Messwellenlänge bei Bestimmung der Farbstabilität beträgt 550 nm, die Temperatur 25 °C. Der Verlauf der Extinktion wird über 12 Stunden verfolgt, die Messungen erfolgen stündlich.

Die Angabe der Farbstabilität erfolgt in Prozent und bezieht sich auf die Abweichung des Messignals bei der jeweiligen Zeit zum anfänglichen Messignal, welches 2 Minuten nach Zugabe von Wasserstoffperoxid aufgenommen wurde.

In der Tabelle ebenfalls mit angegeben sind die $\lambda_{max}$- und $\varepsilon$-Werte des anfänglichen Messignals.

Tabelle

| Kupplungskomponente | | EHT | | EMAE | | F-EMAE | |
|---|---|---|---|---|---|---|---|
| Farbkuppler | | TAP | 4-AAP | TAP | 4-AAP | TAP | 4-AAP |
| $\lambda_{max}$ (nm) | | 552 | 550 | 555 | 552 | 556 | 552 |
| $\varepsilon$ ($cm^2$/mol $H_2O_2$) | | 14,4 | 14,7 | 15,4 | 15,9 | 29,5 | 27,8 |
| Farbstabilität in % nach | 1 h | 0,0 | −0,2 | 0,0 | −0,2 | −0,6 | −2,1 |
| | 2 h | −0,2 | −0,3 | −0,5 | −0,9 | −1,0 | −3,3 |
| | 6 h | −1,4 | −5,5 | −1,2 | −6,6 | −2,2 | −9,6 |
| | 12 h | −5,6 | −13,6 | −3,9 | −15,7 | −5,1 | −17,9 |

**Patentansprüche für die Vertragsstaaten: BE, CH, DE, FR, GB, IT, LI, LU, NL, SE**

1. Aminopyrazolinone der allgemeinen Formel

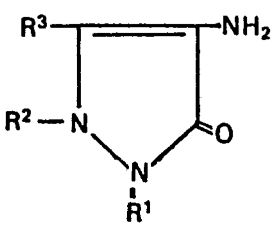

in der $R^1$, $R^2$ und $R^3$ unabhängig voneinander je eine geradkettige oder verzweigte Alkylgruppe mit 1 bis 6 C-Atomen bedeuten und zusammen höchstens 8 C-Atome enthalten.

2. 4-Amino-1,2,3-trimethylpyrazolin-5-on.

3. Verfahren zur Herstellung einer Verbindung nach Anspruch 1 oder 2, dadurch gekennzeichnet, dass man ein 1,2,3-Trialkylpyrazolin-5-on nitriert und die Nitrogruppe dann reduziert.

4. Verwendung einer Verbindung nach Anspruch 1 oder 2 als Farbkuppler zum $H_2O_2$-Nachweis mit einer phenolischen oder anilinischen Kupplungskomponente.

5. Verwendung nach Anspruch 4 mit N-Ethyl-N-hydroxyethyl-3-methylanilin oder N-Ethyl-N-(3-methylphenyl)- N'-acetamidoethylendiamin als anilinische Kupplungskomponente für den Zweck von Anspruch 4.

**Patentansprüche für den Vertragsstaat: AT**

1. Verfahren zur Herstellung eines Aminopyrazolinons der allgemeinen Formel

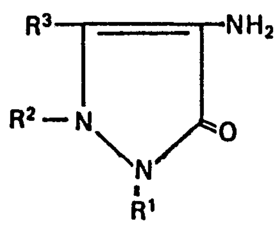

in der $R^1$, $R^2$ und $R^3$ unabhängig voneinander je eine geradkettige oder verzweigte Alkylgruppe mit 1 bis 6 C-Atomen bedeuten und zusammen höchstens 8 C-Atome enthalten, dadurch gekennzeichnet, dass man ein entsprechendes 1,2,3-Trialkylpyrazolin-5-on nitriert und die Nitrogruppe dann reduziert.

2. Verwendung einer Verbindung nach Anspruch 1 als Farbkuppler zum $H_2O_2$-Nachweis mit einer phenolischen oder anilinischen Kupplungskomponente.

3. Verwendung nach Anspruch 2 mit N-Ethyl-N-hydroxyethyl-3-methylanilin oder N-Ethyl-N-(3-methylphenyl)- N'-acetamidoethylendiamin als anilinische Kupplungskomponente für den Zweck von Anspruch 2.

**Revendications pour les Etats contractants: BE, CH, DE, FR, GB, IT, LI, LU, NL, SE**

1. Aminopyrazolinones répondant à la formule générale

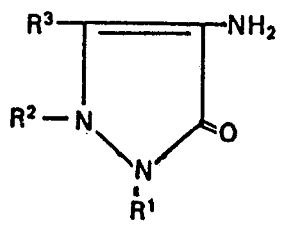

dans laquelle $R^1$, $R^2$ et $R^3$ désignent indépendamment les uns des autres chacun un gropue alkyle linéaire ou ramifié en $C_1$ à $C_6$ et contiennent ensemble 8 atomes C au maximum.

2. 4-amino-1,2,3-triméthylpyrazolin-5-one.

3. Procédé de préparation d'un composé suivant les revendications 1 ou 2, caractérisé en ce que qu'on nitre une 1,2,3-trialkylpyrazolin-5-one et en ce qu'on réduit ensuite le groupe nitro.

4. Utilisation d'un composé suivant la revendication 1 ou 2 comme copulant pour la détection d'$H_2O_2$ avec un constituant de copulation phénolique ou anilinique.

5. Utilisation suivant la revendication 4 avec la N-éthyl-N-hydroxyéthyl-3-méthylaniline ou la N-éthyl-N-(3-méthylphényl)-N'-acétamidoéthylènediamine comme constituants de copulation aniliniques pour le but de la revendication 4.

**Revendications pour l'Etat contractant: AT**

1. Procédé de préparation d'une aminopyrazolinone répondant à la formule générale

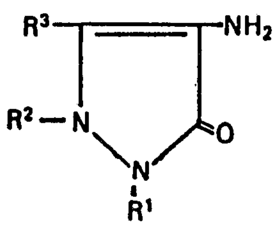

dans laquelle $R^1$, $R^2$ et $R^3$ désignent indépendamment les uns des autres chacun un groupe alkyle linéaire ou ramifié en $C_1$ à $C_6$ et contiennent ensemble 8 atomes C au maximum, caractérisé en ce qu'on nitre une 1,2,3-trialkylpyrazolin-5-one appropriée puis en ce qu'on réduit le groupe nitro.

2. Utilisation d'un composé suivant la revendication 1 comme copulant pour la détection d'$H_2O_2$ avec un constituant de copulation phénolique ou anilinique.

3. Utilisation suivant la revendication 2 avec la N-éthyl-N-hydroxyéthyl-3-méthylaniline ou la N-éthyl-N-(3-méthylphényl)-N'-acétamidoéthylènediamine comme constituants de copulation aniliniques pour le but de la revendication 2.

**Claims for the contracting states: BE, CH, DE, FR, GB, IT, LI, LU, NL, SE**

1. Aminopyrazolinones of the general formula

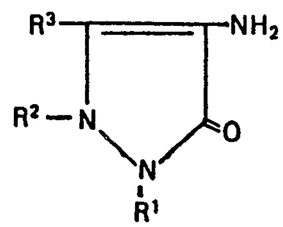

wherein $R^1$, $R^2$ and $R^3$ independently of one another each represent a straight-chain or branched alkyl group having 1 to 6 carbon atoms and together contain a maximum of 8 carbon atoms.

2. 4-Amino-1,2,3-trimethylpyrazolin-5-one.

3. A method for the preparation of a compound according to claim 1 or 2, characterised in that a 1,2,3-trialkyl-pyrazolin-5-one is nitrated and the nitro group is then reduced.

4. Use of a compound according to claim 1 or 2 as a colour coupler for $H_2O_2$ detection with a phenolic or anilinic coupling component.

5. Use according to claim 4 with N-ethyl-N-hydroxyethyl-3-methylaniline or N-ethyl-N-(3-methylphenyl)-N'-acetamidoethylenediamine as an anilinic coupling component for the purpose of claim 4.

**Claims for the contracting state: AT**

1. A method for the preparation of an aminopyrazolinone of the general formula

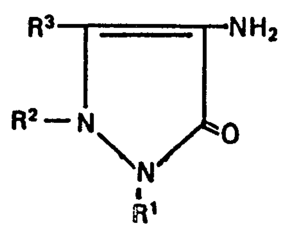

wherein $R^1$, $R^2$ and $R^3$ independently of one another each represent a straight-chain or branched alkyl group having 1 to 6 carbon atoms and together contain a maximum of 8 carbon atoms, characterised in that a corresponding 1,2,3-trialkylpyrazolin-5-one is nitrated and the nitro group is then reduced.

2. Use of a compound according to claim 1 as a colour coupler for $H_2O_2$ detection with a phenolic or anilinic coupling component.

3. Use according to claim 2 with N-ethyl-N-hydroxyethyl-3-methylaniline or N-ethyl-N-(3-methylphenyl)-N'-acetamidoethylenediamine as an anilinic coupling component for the purpose of claim 2.